# EUROPEAN PATENT SPECIFICATION

(11) **EP 2 819 715 B1**
(45) Date of publication and mention of the grant of the patent: **19.07.2017**
(21) Application number: 13708324.2
(22) Date of filing: 26.02.2013
(51) Int. Cl.: A61L 31/02, A61L 31/16

(54) **ANODIZED TITANIUM DEVICES AND RELATED METHODS**
ANODISIERTE TITANVORRICHTUNGEN UND VERWANDTE VERFAHREN
DISPOSITIFS EN TITANE ANODISÉ ET PROCÉDÉS ASSOCIÉS

(30) Priority: 02.03.2012 US 201261606152 P
(43) Date of publication of application: 07.01.2015
(73) Proprietor: Synthes GmbH, 4436 Oberdorf (CH)
(72) Inventor: DISEGI, John, West Chester, PA 19380 (US)
(74) Representative: Klunker IP Patentanwälte PartG mbB
(86) International application number: PCT/US2013/027737
(87) International publication number: WO 2013/130431

(56) References cited:
- EP-A1- 2 180 083
- WO-A1-2010/050401
- US-A1- 2009 093 881
- US-A1- 2011 313 539

## Description

### TECHNICAL FIELD

The present disclosure relates to the field of biomechanical implants and to the field of anodized metals.

### BACKGROUND

Because of its high strength, low weight, and corrosion resistance, titanium has application to various medical implant applications. Because unwanted microbial growth is a concern in medical implant technology, some have attempted to construct titanium implants that feature titanium oxide coatings. Such coatings, however, suffer from poor adhesion to the underlying implant structure, are prone to delamination, and are also associated with a significant reduction in fatigue strength. Accordingly, there is a long-felt need in the art for titanium implant structures that have antimicrobial properties that do not suffer from the drawbacks of titanium oxide coated implant materials. There is also a related need in the field for related methods of fabricating such implants.

EP 2 180 083 A1 discloses a medical implant, such as a dental implant, made of titanium or a titanium alloy. The surface of the implant is electrochemically treated, in particular anodized using an acid, to create a titanium oxide film. The surface treatment is adapted to create a specific color of the implant, such as pink, to adapt the color of the implant to the surrounding tissue. The implant also has antimicrobial properties. Medical implants with antimicrobial properties are also disclosed in US 2009/0093881 A1 and US 2011/0313539 A1. The surface of the titanium implant may be treated to create a film having a porous or nonporous structure. Additional treatments may be added to the surface, such as iodine treatment.

### SUMMARY

This invention relates to a kit comprising first and second medical devices as defined by claim 1, and a method of processing a first and second medical device as defined by claim 11, where at least a portion of the titanium oxide film surmounting at least a portion of the titanium substrate is of an anathase phase and of a selected thickness.

The surface oxide film is treated to transform the surface structure or to optimize the percentage (%) anatase in the surface film. Anatase titanium oxide demonstrates antimicrobial properties when activated under specific photocatalytic conditions. Antimicrobial activation of the anatase titanium oxide is performed in the near ultraviolet wavelength of 350 to 380 nm to create reactive oxygen species and hydroxyl radicals that provide antimicrobial properties. The titanium implant may be activated before the titanium implant is packaged or, alternatively, may be activated in the operating room before implantation using a suitable light source.

A further advantage of the disclosed methods and implants is the ability to provide color coded titanium implants. This color coding may be used to construct an implant system (e.g., color-coded by size, shape, by application, or even by patient type) that also features antimicrobial properties when activated. The anodized film may be thin (in the nanometer range), and because the film is produced by electrochemical oxidation, the anatase film is extremely adherent, durable, and exhibits negligible reduction in fatigue strength for the implant.

### BRIEF DESCRIPTION OF THE DRAWINGS

The summary, as well as the following detailed description, is further understood when read in conjunction with the appended drawings. For the purpose of illustrating the invention, there are shown in the drawings exemplary embodiments of the invention; the drawings are not necessarily drawn to scale or proportion. In the drawings:
Figure 1 illustrates the x-ray crystallography spectrum for an exemplary anatase-phase material according to the present disclosure, showing the anatase phase present in the material;
Figure 2 illustrates an exemplary setup for fabricating an anatase film on the surface of a titanium substrate;
Figure 3 illustrates several colored anatase samples according to the present disclosure;
Figure 4 illustrates an additional composite image of the different voltage levels we tested in a 0.5 molar sulfuric acid bath with a square wave (DC);
Figure 5 illustrates x-ray diffraction data for the samples of Figure 4, with the anatase peak labeled;
Figure 6 presents the same x-ray diffraction data with the rutile peak labeled;
Figure 7 illustrates a composite image of samples tested in a 0.94 molar sulfuric acid bath (square wave DC);
Figure 8 presents x-ray diffraction data from the samples of Figure 7 with the anatase peak labeled; and
Figure 9 presents x-ray diffraction data from sputter-coated materials as compared to a sample (top graph) according to the present disclosure, with the anatase peak in the topmost sample labeled.
Figure 10 presents an SEM image showing the natural forming oxide of titanium;
Figure 11 presents an SEM image showing the titanium oxide after pickling in an exemplary nitric-hydrofluoric acid solution;
Figure 12 presents a comparatively low magnification SEM image of a gold anodized titanium sample tested in 0.5 M H2SO4;
Figure 13 presents a comparatively higher magnification SEM image of a gold anodized titanium sample tested in 0.5 M H2SO4;
Figure 14 presents a higher magnification SEM image of a gold anodized titanium sample tested in 0.5 M H2SO4;
Figure 15 presents a low magnification SEM image of a gold anodized titanium sample tested in 2 M H2SO4;
Figure 16 presents a high magnification SEM image of a gold anodized titanium sample tested in 2 M H2SO4;
Figure 17 presents a high magnification SEM image of a gold anodized titanium sample tested in 2 M H2SO4;
Figure 18 presents a low magnification SEM image of a green anodized titanium sample tested in 0.94 M H2SO4;
Figure 19 presents a high magnification SEM image of a green anodized titanium sample tested in 0.94 M H2SO4;
Figure 20 presents a high magnification SEM image of a green anodized titanium sample tested in 0.94 M H2SO4;
Figure 21 presents a low reproduction SEM image obtained with the EBSD detector showing the area being scanned of the 0.94 M green anodized titanium;
Figure 22 presents a grain orientation map and associated inverse pole figure map for the 0.94 M green anodized titanium;
Figure 23 presents an EBSD image showing the crystalline phases detected and associated area fractions for the 0.94 M green anodized titanium;
Figure 24 presents an x-Ray diffraction scan of a green anodized titanium sample tested in 2 M H2SO4;
Figure 25 presents a low magnification SEM image of a green anodized titanium sample tested in 2 M H2SO4;
Figure 26 presents a high magnification SEM image of a green anodized titanium sample tested in 2 M H2SO4;
Figure 27 presents a high magnification SEM image of a green anodized titanium sample tested in 2 M H2SO4;
Figure 28 presents an SEM image obtained with the EBSD detector showing the area being scanned of the 2 M green anodized titanium;
Figure 29 presents an EBSD image showing the grain orientations and associated inverse pole figure map for the 2 M green anodized titanium;
Figure 30 presents an EBSD image showing the crystalline phases detected and associated area fractions for the 2 M green anodized titanium; and
Figure 31 presents an x-Ray diffraction scan of a green anodized titanium sample tested in 2 M H2SO4.

### DETAILED DESCRIPTION OF ILLUSTRATIVE EMBODIMENTS

In a first aspect, the present disclosure provides medical devices. These devices may be configured as, e.g., implants, supports, fasteners, and the like.

The medical devices first include a substrate comprising titanium. The substrate may be solid titanium (e.g., a solid titanium rod, sheet, plate, and the like), but may also include a titanium coating or shell associated with a core material. As one example, the device may include a core that is surmounted by a titanium (pure, alloy, or even composite) coating. The titanium coating may be bonded to the core or mechanically affixed or otherwise interlocked with the core. A device according to the present disclosure may feature an exterior that has a region of titanium, titanium alloy, or of titanium composite, and another region that is free of titanium. Such devices are suitable for applications where the titanium-bearing portion is implanted into a subject's body, and the non-titanium bearing portion lies outside of the subject.

The core may be polymeric or other material (e.g., metal) that is adaptable to use in medical implants. Exemplary polymers include PEEK, PEKK, UHMWPE, poyphenylsulfone, HDPE, PCU, and the like. PE, PP, and PC may also be used.

The devices include a titanium oxide film that surmounts at least a portion of the titanium of the device, with at least a portion of the titanium oxide film being anatase phase. The film may be anodized in form. The titanium oxide film is of such a thickness so as to impart a visually perceptible color to the medical device.

The substrate may, as described above be essentially pure titanium. The substrate may be solid titanium (e.g., a solid rod, plate, or platelet). Alternatively, the substrate may comprise a titanium alloy. Virtually any implantable titanium alloys may be used in the disclosed devices. A partial, nonexhaustive listing of such alloys includes, e.g., Ti6Al7Nb, Ti6Al4V, Ti6Al4V ELI, Ti15Mo, Ti13Nb13Zr, Ti3Al2.5V, and Ti12Mo6Zr2Fe. The implantable alloys may be anodized and will suitably contain a % of anatase in the mixed oxide film. For example, an anodized Ti6Al7Nb substrate is comprised of titanium oxide plus aluminum oxide plus niobium oxide and will contain less anatase than a pure titanium substrate. The specific anodizing parameters required to produce an anatase titanium oxide structure will also vary for each alloy and will affect the amount of anatase that is present in the mixed oxide film. The devices may feature apertures (smooth or threaded) to facilitate installation of the devices into a subject. For example, a support plate used to support a broken long bone implant may feature smooth apertures at either end, through which screws or other fasteners may be installed to fix the plate to the long bone. The fasteners themselves may, as described elsewhere herein, feature anatase regions according to the present disclosure so as to render the fasteners antimicrobial. The fasteners may also feature a color that matches that of the support plate so as to indicate to the user that the fasteners are adapted for use with the plate.

The thickness of the film may vary, depending on the needs of the user and the desired color profile. The thickness is suitably in the range of from about range of 20 nm to about 500 nm, or from 100 nm to about 400 nm, or even from about 130 nm to about 275 nm. These thicknesses enable the production of devices that exhibit colors of, e.g., gold, rose red, purple, aqua, and green, among others. Other colors, such as bronze, brown, dark purple, blue, light blue, green-gray, and light green may also be produced by modulating the thickness of the anatase coating.

A device according to the present disclosure may include a first region that features a film of one thickness and another region that features a film of another thickness. In this way, a device may include two or more regions that feature different colors. This may be used so as to inform the user as to the alignment of the device when in use. For example, a device may be configured to have a blue distal region and a green proximal region. The disclosed devices may also be configured such that a colored region on an implant (e.g., blue) coordinate with the fasteners (blue screws, nails, etc.) that are to be used with that implant.

The devices may be configured to serve in a variety of applications. In some embodiments, the devices are adapted to serve as implants. The implants may be suitable for long bone implant purposes or for implantation as other bones. The implants may be configured as plates, strips, ribbons, or the like. Alternatively, the implants may be configured as needles, catheters, cannulas, or even as other instruments such as scoops, rasps, and the like. Implant configurations are considered especially suitable, as such configurations are capable of taking advantage of the antimicrobial characteristics of the disclosed materials. The disclosed devices may also be applied as total joints (hips, wrists, shoulders, ankles, knees, spinal disc prostheses, arthoplasty devices, and the like). The disclosed devices may also be applied as plates, screws, pins, intramedullary nails, neurological implants, mandibular implants, mid-face implants, spinal rods, spinal clamps, intervertebral cages, and the like.

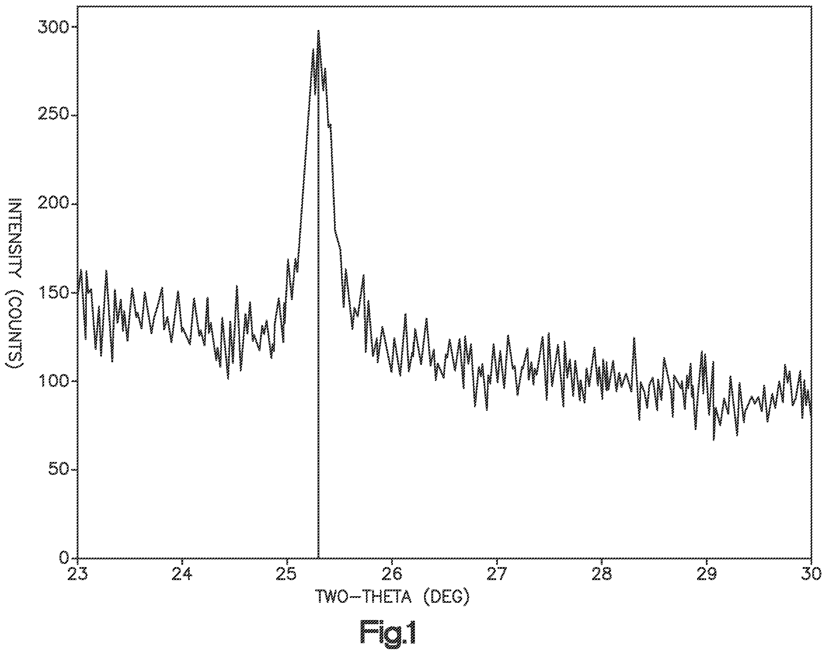

The films of the disclosed devices suitably comprise a content that is suitably more than 95% anatase for commercially pure ("CP") titanium. The film may be more than about 5%, 15%, 25%, 35%, 45%, 55%, 65%, 75%, 85% anatase phase. In embodiments where titanium alloys are used, the device film may be less than 95% anatase, depending on the composition of the mixed oxide film composition after anodizing. In certain embodiments, the titanium oxide film includes greater than 95% anatase and less than about 5% rutile phase. Without being bound to any single theory, the anatase titanium oxide film can be described as a cohesive single-phase oxide that exhibits a distinct crystallographic X-Ray structure, as shown in Figure 1, which figure illustrates the anatase phase present in a sample according to the present disclosure.

The substrate may, in some embodiments, be a mixture of a polymer and titanium. Such composites may include a polymer composition combined with titanium or titanium alloy. The polymer component of the substrate may be a single polymer (e.g., PEEK), or multiple polymers (e.g., PEEK and PP) or even a copolymer. The substrate may comprise a mixture of titanium bodies (particles, flakes, and the like) dispersed within or on the bulk of a polymer or other matrix. The film may be integral to the device.

Also provided are methods of fabricating medical devices. These methods include contacting a substrate material comprising titanium with an electrolyte and anodizing the substrate material by exposing the substrate material to a voltage so as to give rise to film of titanium oxide surmounting at least a portion of the substrate material, as defined by claim 11.

The applied voltage is suitably in the range of from about 25 V to about 400 V, or from about 50 V to about 350 V, or from about 200 V to about 250 V. The voltage may be applied in intervals. The voltage may increase over time, or may be applied at a constant level. The voltage may be increased over time. The increase may be linear, exponential, or step-wise. The voltage may also have a sine waveform, square waveform, triangle, or sawtooth waveform.

In one exemplary embodiment, devices were fabricated using a DC rectifier. The voltage was applied with a 10 volt incremental increase every 10 seconds. A programmable square wave waveform was used, with an on-time of 1-5 micro seconds and an off time 99 microseconds. The electrolyte used was a 0.94 M sulfuric acid with a bath pH of about 0.15 at room temperature. Other suitable electrolytes investigated were 0.5M sulfuric acid (pH 0.30), 0.94 M sulfuric acid (pH 0.15), and 2.0 M sulfuric acid (pH -0.30). 6.0 M sulfuric acid is also a suitable electrolyte, as such an electrolyte is capable of producing a comparatively high percentage of anatase in the color anodized film. Electrolytes - e.g., sulfuric acid - at from about 0.3 M to about 7.0 M or even 9.0 M (e.g., 2.8 M, 3.8 M, 5.6 M, and 6.0 M) are considered especially suitable for the disclosed techniques.

The electrolyte may be a salt solution, or an acid solution. Various salts (sodium chloride, calcium chloride, and the like) may be used. Various acids may be used in the electrolyte, such as acetic, citric, nitric, sulfuric, and other acids may be used. An electrolyte may, for example, comprise a mixture of ACS grade nitric acid (67-70%) and distilled water.

The user may clean or otherwise pretreat ("pickle") the titanium before processing, as desired. A variety of methods may be used to clean the titanium. For example, one may clean the titanium by scrubbing or brushing with a wire or other brush. Grinding, draw filing, and acid picking may also be used. Various combinations of nitric acid plus hydorfluoric acid may be used as long as the volume % nitric acid to volume % hydrofluoric acid ratio is greater than 10:1 to minimize the occurrence of hydrogen embrittlement. One may also use a water rinse to remove acid, followed by a hot water rinse to facilitate drying. Another exemplary pretreatment can be a nitric acid-hydrofluoric acid solution (e.g., 20:2 ratio). Immersion in nitric-hydrofluoric acid solution is used to clean and activate the titanium surface before electrolytic anodization. The ratio of nitric acid to hydrofluoric acid may be adjusted so as to avoid hydrogen pickup in the titanium material. A nitric acid to hydrofluoric acid ratio of minimum ca. 10 to 1 to minimize hydrogen absorption during acid treatment is recognized in ASTM B600 Standard Guide for Descaling and Cleaning Titanium and Titanium Alloy Surfaces.

A user may also apply an activation process to configure the titanium film for antimicrobial activity. Without being bound to any single theory, anatase activation may be effected by the near ultraviolet wavelength of 350 to 380 nanometers so as to create reactive oxygen species and hydroxyl radical that provide antimicrobial properties.

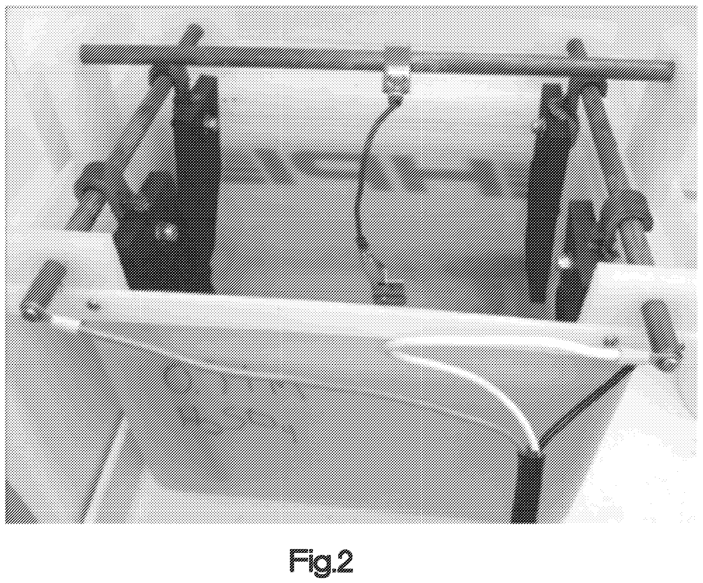

In one exemplary embodiment, titanium implants or coupons are cleaned in an alkaline bath or detergent to remove oil, cutting fluid, and other loose surface contaminants. The implants are then immersed in a nitric acid-hydrofluoric acid pre-treatment solution (e.g., 20:2 ratio). Implants are then placed in a titanium basket or a clamping device in contact with a copper bus bar that connected to a DC rectifier power supply, as illustrated in Figure 2. The clamped implant or basket was immersed in a 0.94 M sulfuric acid electrolyte and the voltage was increased in 10 volt incremental increase every 10 seconds.

As shown in Figure 2, a power supply may be connected through the negative lead (anode) to conductive (e.g., copper) bars running across the short lengths (side to side) of the anodizing bath to carbon counter electrodes. The exemplary negative lead shown here is split into two cables for this setup with two carbon counter electrodes on each anode copper bar. The power supply is also connected through the positive (cathode) lead to a copper bar that runs across the length of the anodizing bath as shown in Figure 2. The positive lead is connected directly to the cathode copper bar and the samples are in turn connected through a metallic clamp, and the samples are then suspended in the electrolyte.

The carbon counter electrodes are spaced out evenly from one another in order to give the most efficient anode to cathode area in the electrolyte (the most efficient flow of electrons in solution). Alternatively, the positive lead from the power supply could be connected to the cathode bar(s) and the negative lead connected to the anode bar(s). The power (voltage and amperage) of the power supply, number and spacing of counter electrodes, and number of cathode bars would depend on the size of the anodizing bath.

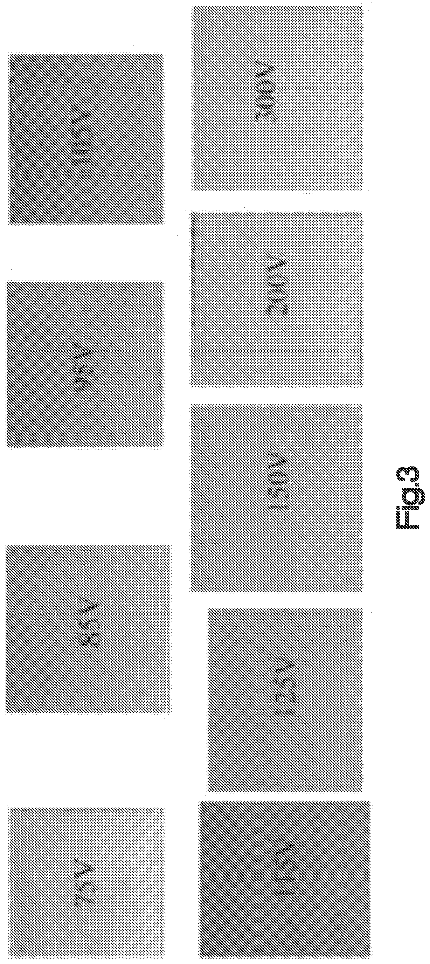

Processed titanium coupons are shown in Figure 3. At the upper left of the figure, a coupon with a yellow-gold color is shown. This coupon was produced by processing at 75 V. The second coupon from the upper left exhibited a pink-rose color, and was processed at 85 V. The third coupon from the upper left exhibited a violet color, which coupon was processed at 95 V. The coupon fourth from the upper left (processed at 105 V) exhibited an aqua blue color. The coupons in the lower row, from left to right respectively, exhibited blue (115 V), blue-green (125 V), medium green (150 V), green (200 V), and light green (300 V) colors.

The foregoing samples were produced using a waveform with a time increment step size of 10 seconds and a voltage step size of 5, 10 or 20V; other voltage steps of from 0.01 V to 50 V are also suitable. The voltage step size was limited to the inputs on the current power supply which had only 15 steps available; this should not be understood as limiting the present disclosure in any way. Thus, the final voltages of 150V and less were increased at 10V every 10 seconds and final voltages of > 150V are stepped up at 20V every 10 seconds. Also any final voltage not an integer of 10 had a 5V end step for 10 seconds. For example, a final voltage of 70V would have a recipe of 10V 10sec, 20V 10sec, 30V 10sec, 40V 10sec, 50V 10sec, 60V 10sec, and 70V 10sec. A 75V final voltage would have a recipe of 10V 10sec, 20V 10sec, 30V 10sec, 40V 10sec, 50V 10sec, 60V 10sec, 70V 10sec, and 75V 10sec. Another example is for a final voltage of 200V which is 20V 10sec, 40V 10sec, 60V 10sec, 80V 10sec, 100V 10sec, 120V 10sec, 140V 10sec, 160V 10sec, 180V 10sec, and 200V 10 sec. The 10 second durations of these voltages is not limiting, as voltages may be applied for from about 0.01 seconds to about 10, about 20, about 30, about 60, about 120, about 300, or even about 500 seconds.

The output color is related to the thickness of the surface oxide created. The oxide layer created depends on the final voltage applied, the area of the sample exposed to the electrolyte (current density, A/cm²), and also sample surface condition. Current for the exemplary system was set at 10 amps and the area suspended in the electrolyte was constant for all samples. Further, all samples were prepared for anodization using the same techniques previously described. Therefore, the only variable that changed color (oxide thickness) was the final applied voltage. Without being bound to any particular theory, exposure time at the final voltage may noe necessarily change (purple to green for example) the final color of the surface oxide and will be in the range of the corresponding thickness values given in the following table, which table relates exemplary surface oxide thicknesses (given in nm) to surface color appearance:
Bronze: 10-25
Brown: 25-40
Dark Purple: 40-50
Blue: 50-65
Light Blue: 75-100
Green Gray: 100-115
Light Green: 110-125
Gold: 135-150
Rose Red: 150-165
Purple: 160-200
Aqua: 230-250
Green: 250-275

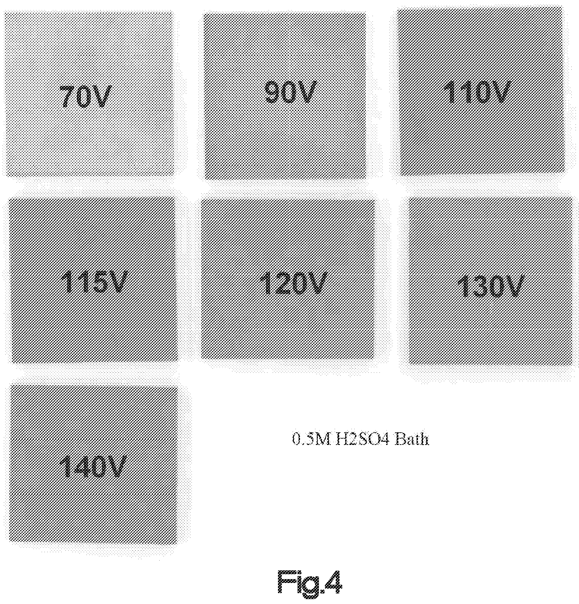

Additional, exemplary samples are shown in Figure 4. The samples in that figure were as follows: 70V (yellow-green), 90V (pink-rose), 110V (blue), 115 (blue-violet), 120V (green), 130V (green), 140V (medium green).

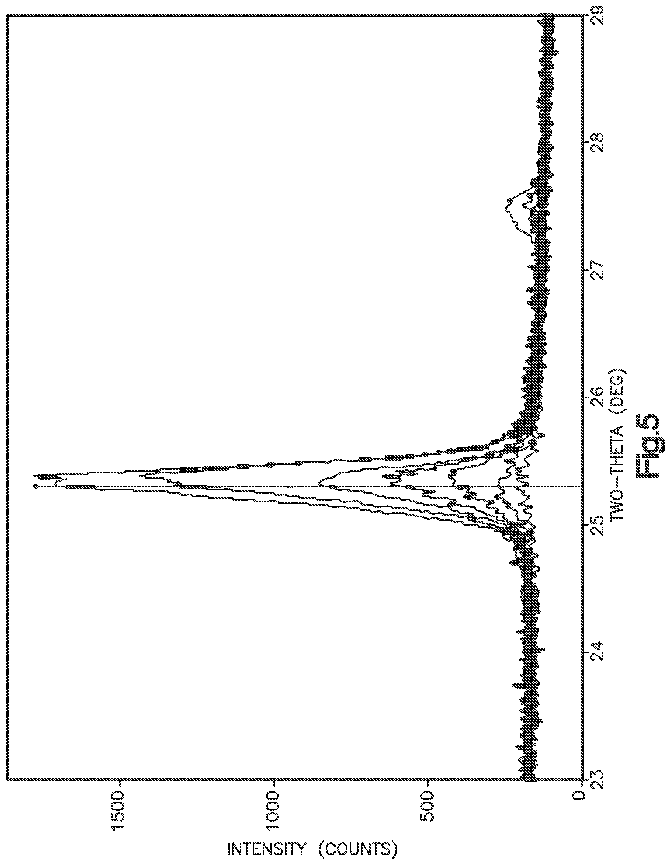

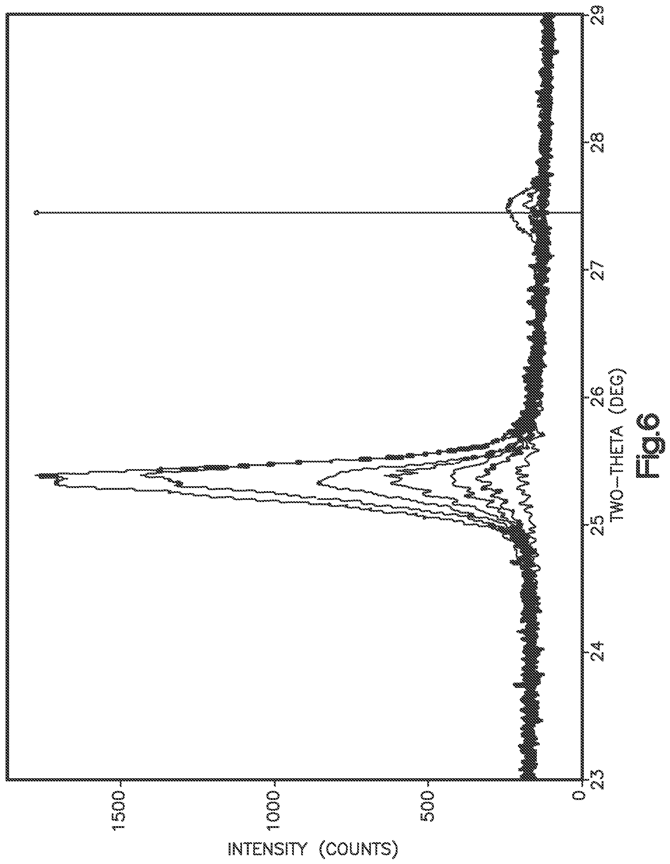

Figure 5 presents x-ray diffraction spectra for the samples shown in Figure 4. As shown in the figure, each of the samples presents a characteristic anatase peak at a two-theta value of about 25.25 degrees. Figure 6 presents x-ray diffraction data for the samples shown in Figure 4 and Figure 5, with the location of the characteristic rutile peak (not present in the samples) labeled.

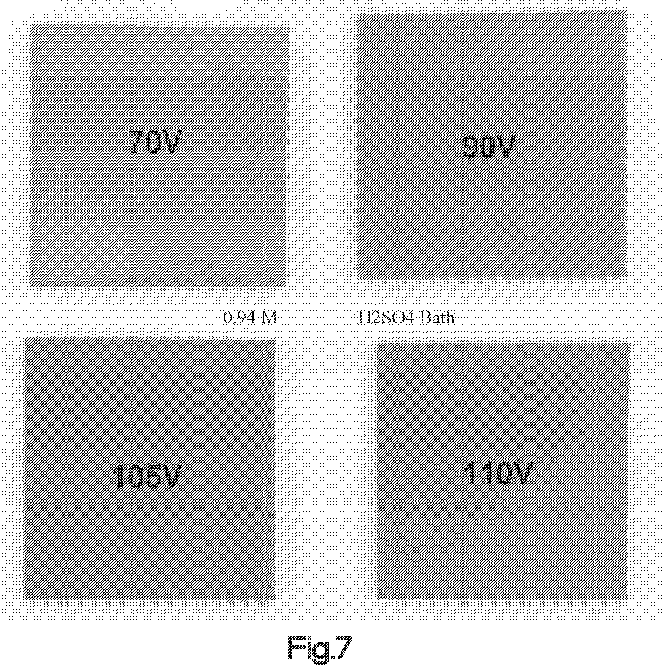

Figure 7 illustrates a composite image of samples tested in a 0.94 molar sulfuric acid bath, processed with a square wave voltage and a DC rectifier. The samples 70V (green-yellow), 90V (pink-rose), and 105V (blue-rose) exhibit color that varied according to the processing conditions for the samples.

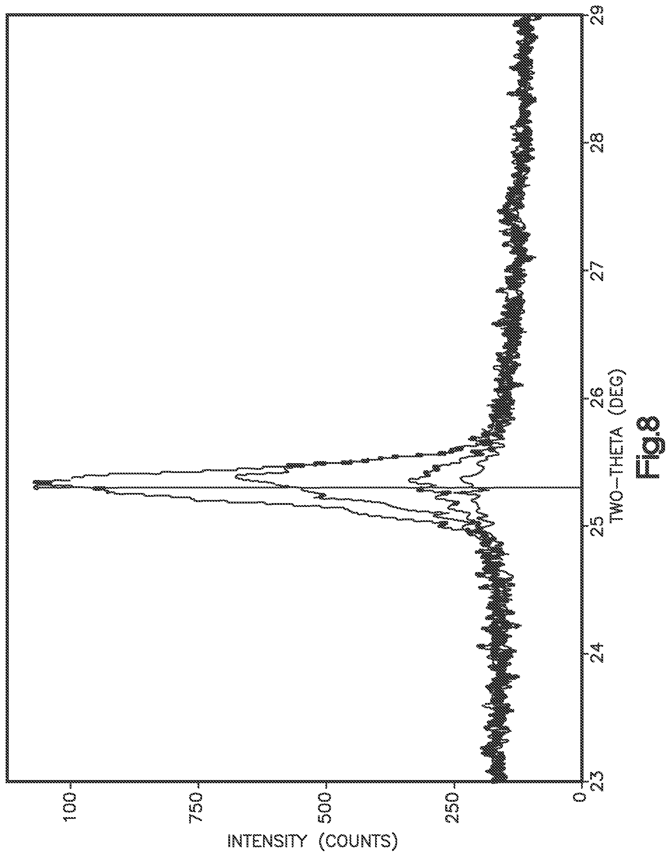

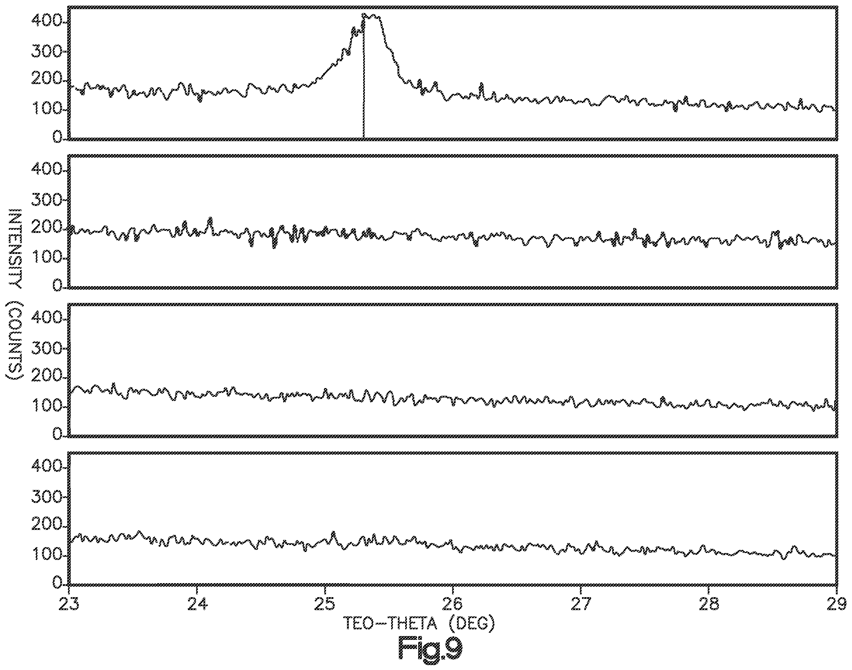

Figure 8 presents x-ray diffraction data from the samples of Figure 7. The anatase peak for the samples is labeled - as shown in the figure, each sample exhibits an anatase peak. Figure 9 presents a x-ray diffraction data for materials according to the present disclosure (uppermost chart) that exhibit a purplish color that is essentially equivalent to the color of vacuum sputter-coated materials (lower charts) which do not contain anatase in the colored oxide film. The sputter-coating may not in all cases demonstrate antimicrobial properties after light activation, as sputter-coated film does not contain an anatase peak..

The present disclosure also provides kits. The disclosed kits include first and a second devices, each of the devices having at least one surface that is at least partially surmounted by a film of titanium oxide, that is at least partially anatase in phase and that confers a visually perceptible color on the devices, the first and second devices differing in visually perceptible color and in at least one other physical characteristic, as defined by claim 1.

As one example, a kit may include multiple implants featuring different colors. For example, the largest implant in the kit may feature a green color, and the smallest implant may feature a gold color. The colors may also be used to distinguish between implants that differ in some other physical characteristic. For example, a kit may include a gold-colored implant adapted for use as a humerus implant, and a rose-colored implant adapted for use as a radius implant. The kits may also include color-coded anchors, nails, or screws that match or approximate the color of the devices with which they are intended to cooperate. Alternatively, fasteners may be color-coded by size, e.g., fasteners of 5 mm diameter are gold-colored, and fasteners of 10 mm diameter are rose-colored.

The kit may also, in some embodiments, include a source of that is operable to emit light of a wavelength and intensity sufficient to cause the titanium dioxide to exhibit a biocidal effect upon irradiation with light from the light source. This light source may be a lamp, a laser, or similar. One exemplary light source is the TL 20W/05 UV lamp from Phillips Co., Holland, operating at about 360 nanometers. The near ultraviolet (NUV) wavelength occurs primarily between 300 nm to 400 nm and the preferred activation wavelength is from about 350 nm to about 380 nm. Fluorescent black lights coated with specific phosphers on the inside of the tube may also be used, such as but not limited to, europium doped strontium flouroborate or europium doped strontium borate (368 nm - 371 nm emission peak) and lead-doped barium silicate (350 nm - 353 nm emission peak). Other ultraviolet wavelengths outside of the preferred anatase activation range such as ultraviolet A (UVA) at 315 nm - 400 nm, ultraviolet B (UVB) at 280 nm - 315 nm, and middle ultraviolet (MUV) at 200 nm - 300 nm may be used. Antimicrobial activation may, in some embodiments, be tuned as a function of light exposure. Other UV arc lamps such as xenon, deuterium, mercury-xenon, and metal-halide provide a continuous emission spectra and are not effective anatase activation sources. The kits may include a removable package that is suitably essentially transparent to ultraviolet light. The first, second, or both devices are suitably disposed within the removable package. The package may be a bag, a box, and the like. The kit may be disposed in a suitcase, box, or other container. As described elsewhere herein, the devices may be exposed to illumination to activate them before being sealed into a package or sealed into a kit.

The user suitably illuminates the implant or other device before implantation, although the devices may be illuminated after installation. Illumination and activation may also be effected after the device is fabricated, or even after the device is packaged. In this way, the fabricator may package the devices in a sterile package (e.g., a bag or box) and then illuminate the device to render it antimicrobial while within the sterile package. In this way, the device may remain sterile until the user removes the package in preparation for device installation. The devices may, alternatively, be illuminated first and then sterilized when in a package or sterilized and then packaged.

### Additional Disclosure

The X-Ray diffraction data generated from some tests show that anatase may form close to the oxide thickness associated with a gold color. In some cases, higher levels, other than green-gray and gray, of crystalline anatase and/or rutile is associated with the oxide thickness associated with a green color. For this reason, gold and green anodized samples were chosen as additional test samples.

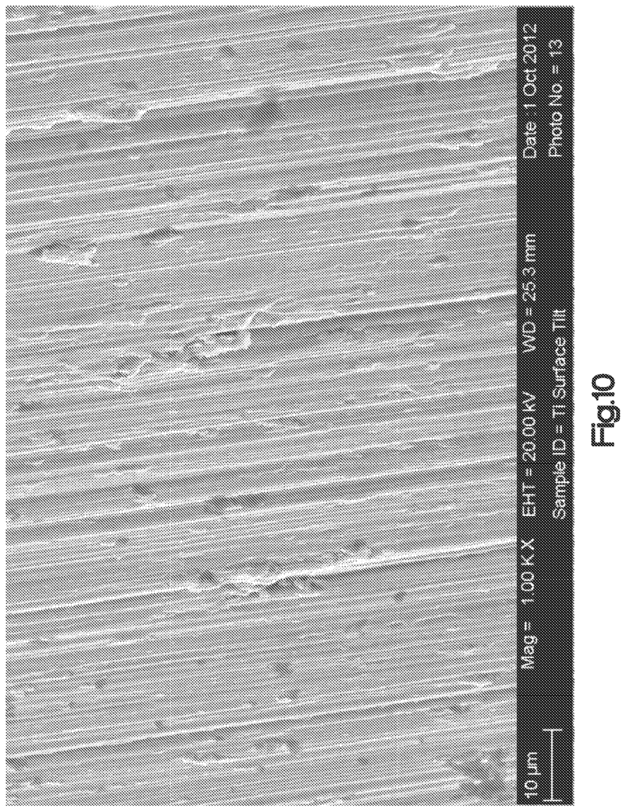

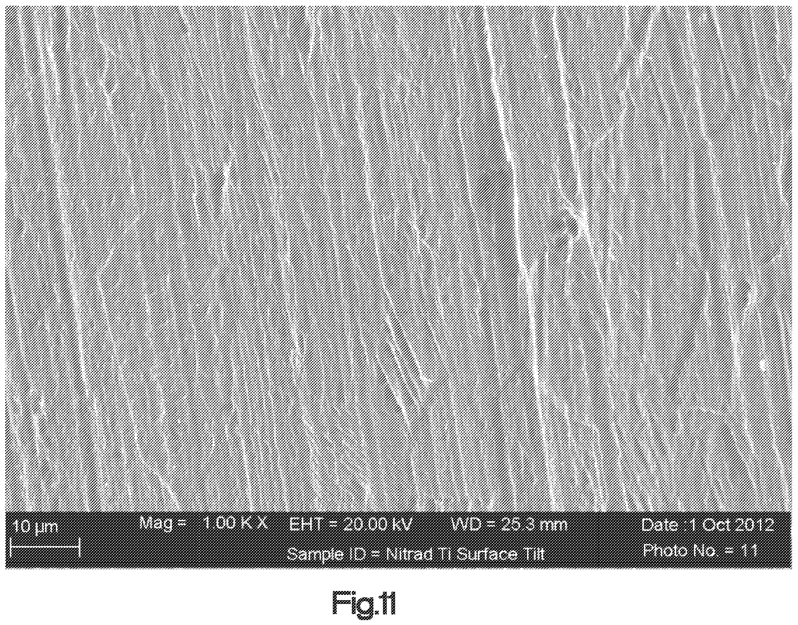

Samples were tilted to an angle of 60-70° in order to detect the surface morphologies using scanning electron microscopy (SEM). Observation of the tilted oxide showed different areas of surface roughness that cannot be distinguished when the samples are flat. Electron backscattered diffraction (EBSD) was used on two green anodized samples to determine if there was a crystalline difference in the different areas observed and the presence and distribution of the crystalline phases if present. In order to establish baseline information, one half of a titanium sample was pickled (nitric-hydrolfluoric solution for 30 seconds) and the other half remained the natural forming surface oxide. SEM images of the natural surface are shown in Figure 10 and the pickled surface in Figure 11. Figure 10 shows a roughened surface from the as rolled titanium sheet, while Figure 11 shows a less roughened surface and etching of the grain boundaries.

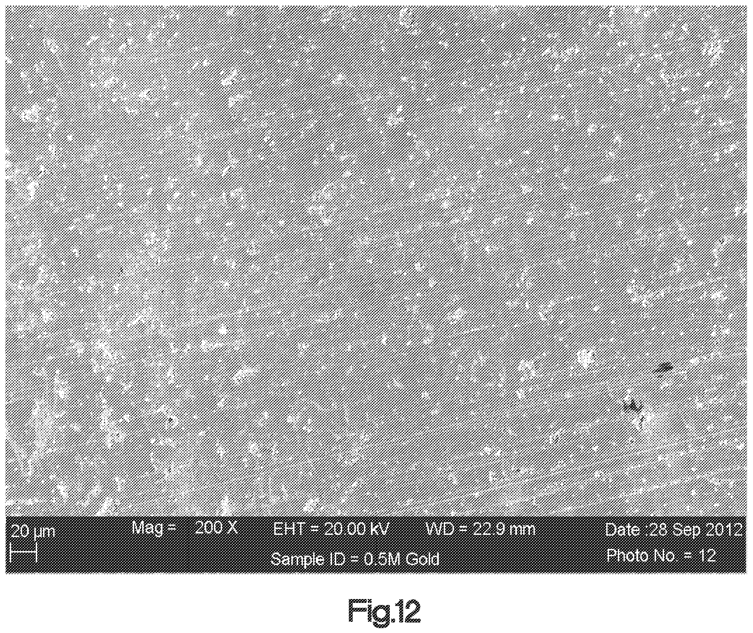

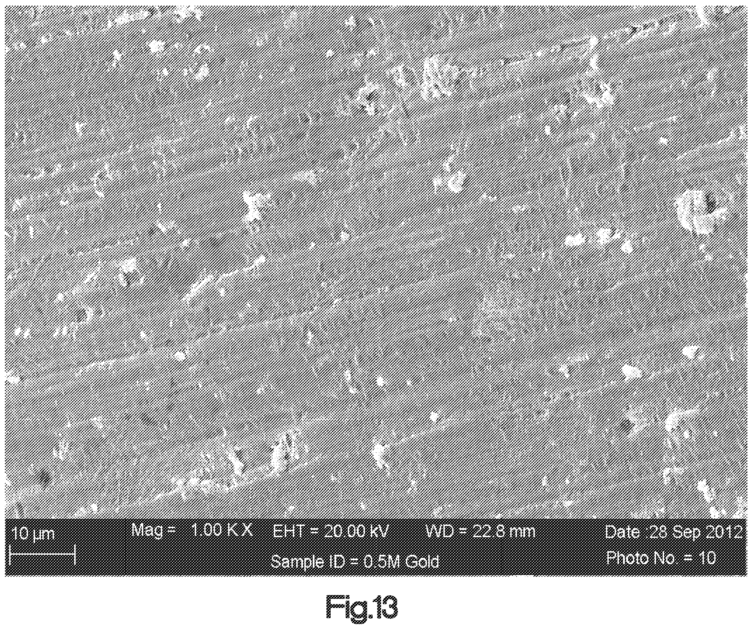

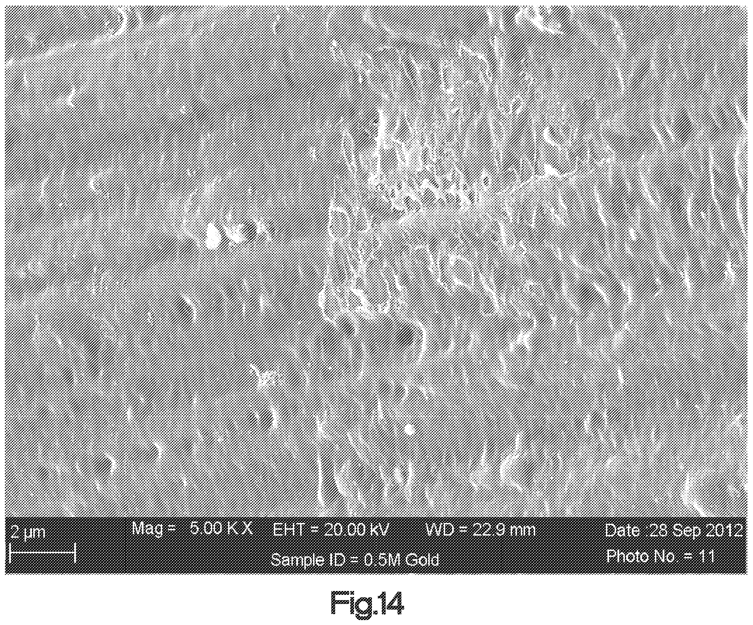

Figures 12-14 show the surface oxide of a gold anodized titanium sample tested in 0.5 M sulfuric acid. The low magnification SEM image (Figure 11) shows a distribution of light and dark colored areas without any discernible surface roughness or morphological differences. Figure 12 shows a higher magnification (1000X) of the same area. No surface difference can be distinguished between the darker and lighter areas and is comparable to the nitric-hydrofluoric pickled surface (Figure 11). Figure 14 shows an even higher magnification (5000X) in which the lighter surface area has some micro porosity forming while the dark area appears to remain smooth. The X-Ray diffraction data did not show any peak intensities for anatase or rutile, indicating that the surface is amorphous or the crystalline areas present have a small intensity that cannot be distinguished from the background.

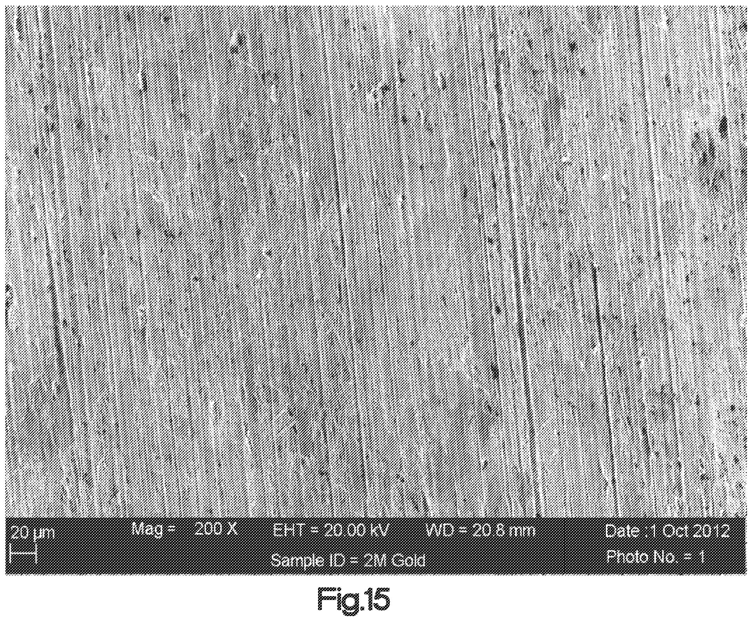

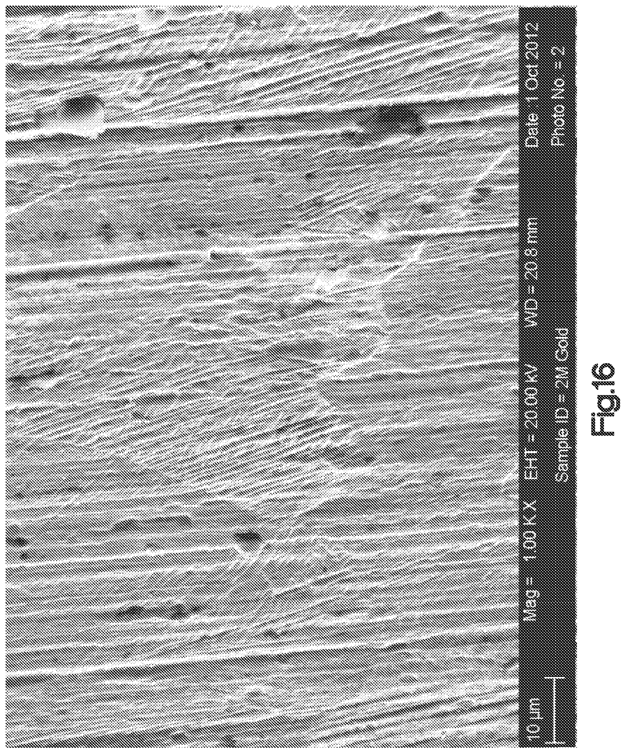

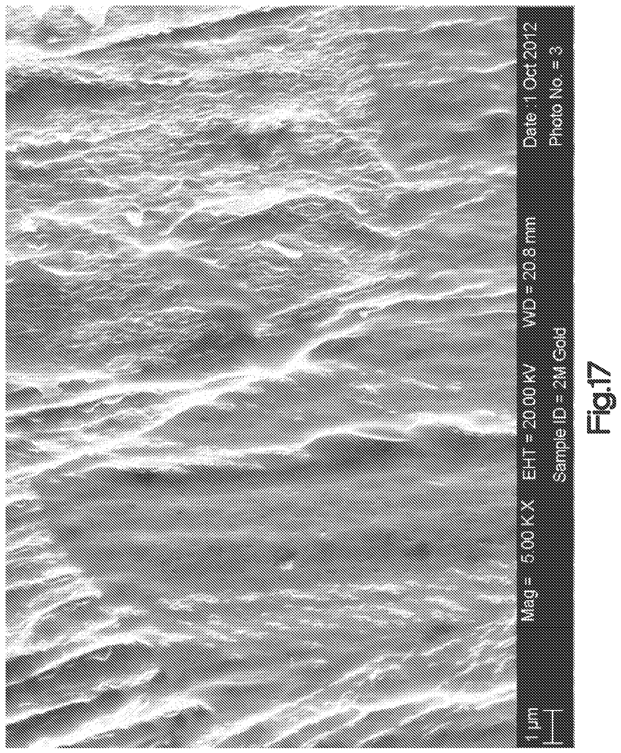

Figures 15-17 show the surface oxide of a gold anodized titanium sample tested in 2 M sulfuric acid. Figure 15 is a low magnification SEM image that shows a distribution of light and dark colored areas comparable to the 0.5 M gold sample (Figure 12). Figure 16 is a higher magnification (1000X) image that shows a few titanium grains with little to no discernible surface morphology difference between the dark and lighter areas. However, a higher magnification (5000X) of the same area shown in Figure 17 shows the lighter area to have a more mirco porous surface morphology compared to the smooth dark area. This morphology difference is similar to that shown in Figure 14 (high magnification 0.5 M gold sample) but seems to cover more of the surface. Again, no anatase or rutile peaks were found in the x-ray diffraction data for this sample.

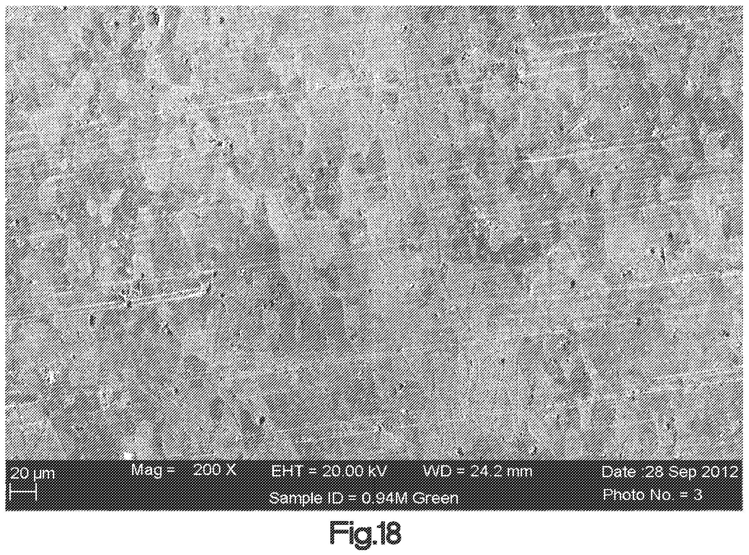

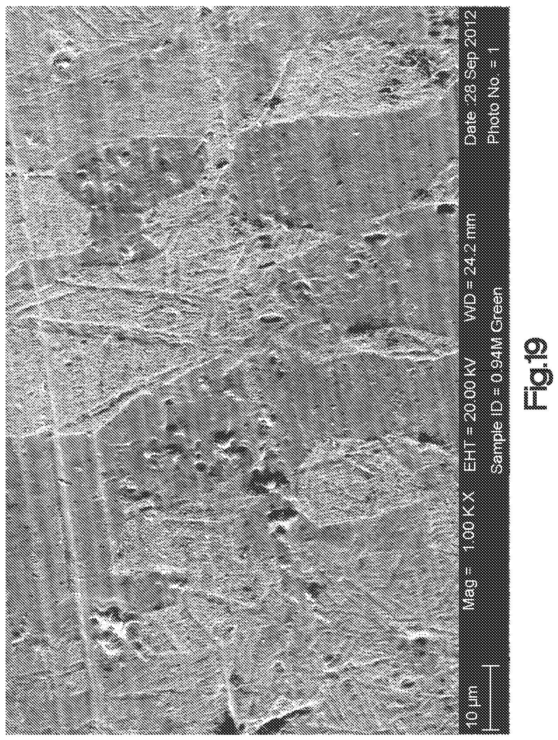

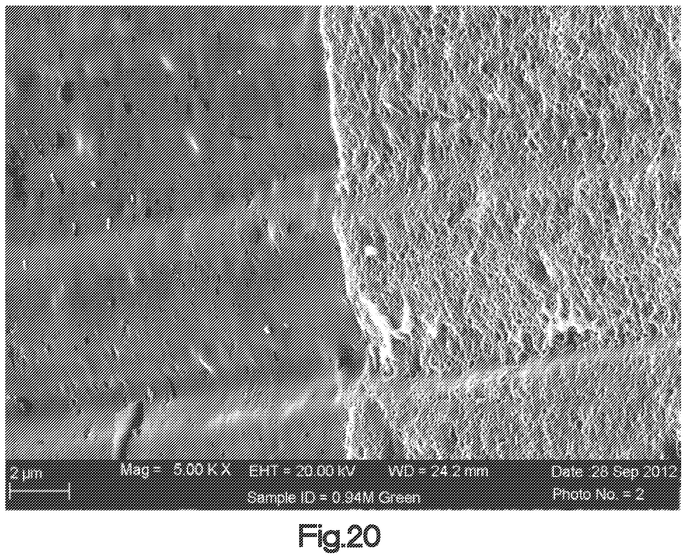

Figures 18-20 show the surface oxide of a green anodized titanium sample tested in 0.94 M sulfuric acid. The low magnification SEM image (Figure 18) shows a distribution of light and dark colored grains without any discernible surface roughness differences. Figure 19 shows a higher magnification (1000X) of the same area. A surface roughness and morphology difference can be clearly seen between the darker smooth areas in the middle of the image compared to the lighter areas around the periphery. Figure 20 shows an even higher magnification (5000X) in which the texture differences can be distinguished as boundaries between smooth flat areas and porous rougher areas. EBSD was used to evaluate the boundary seen in Figure 20 at an approximate magnification of 15,000X. The high magnification was needed to distinguish the very small anatase and rutile grains.

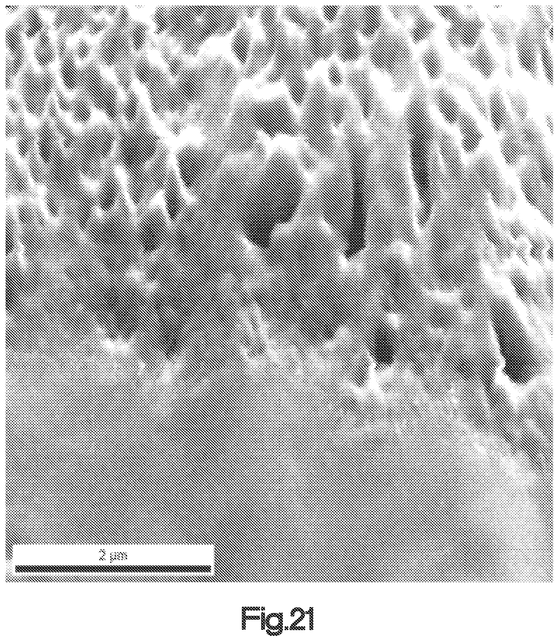

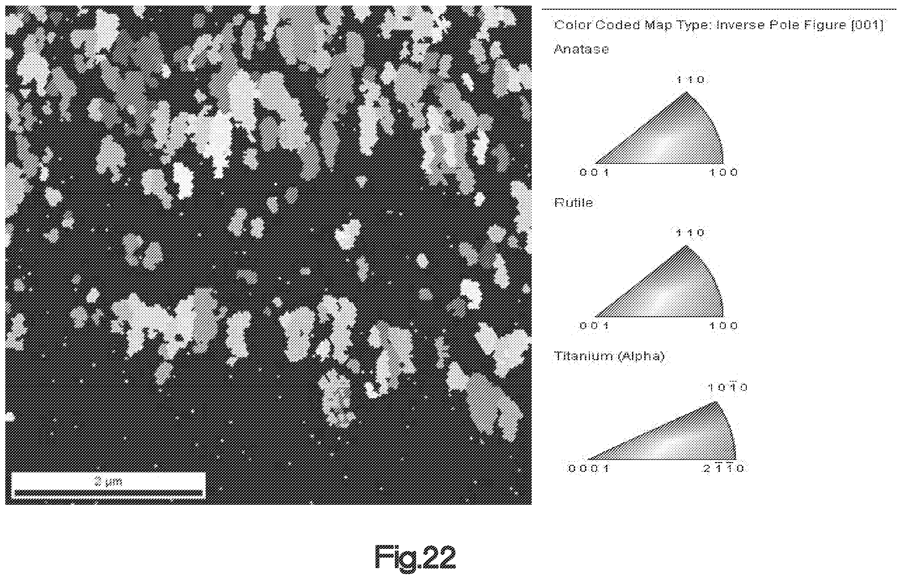

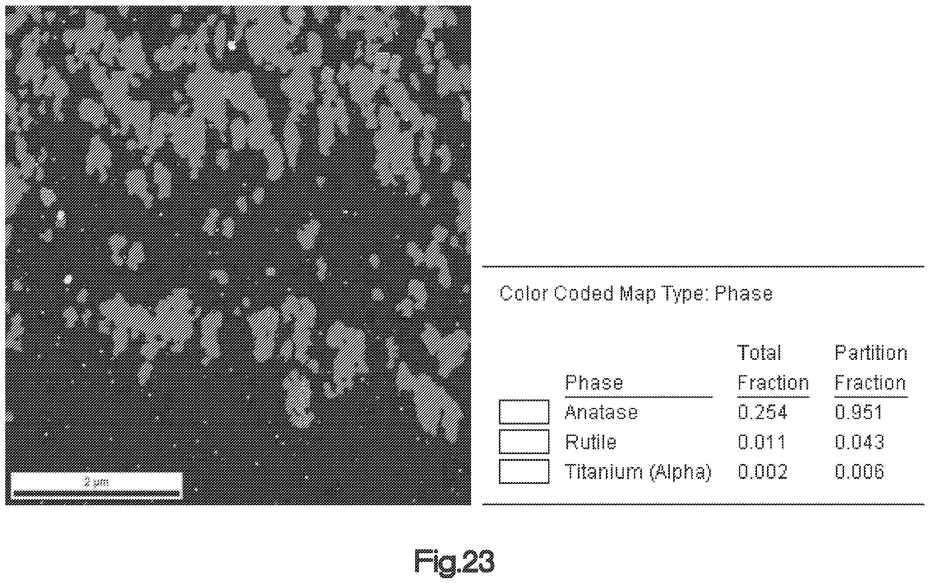

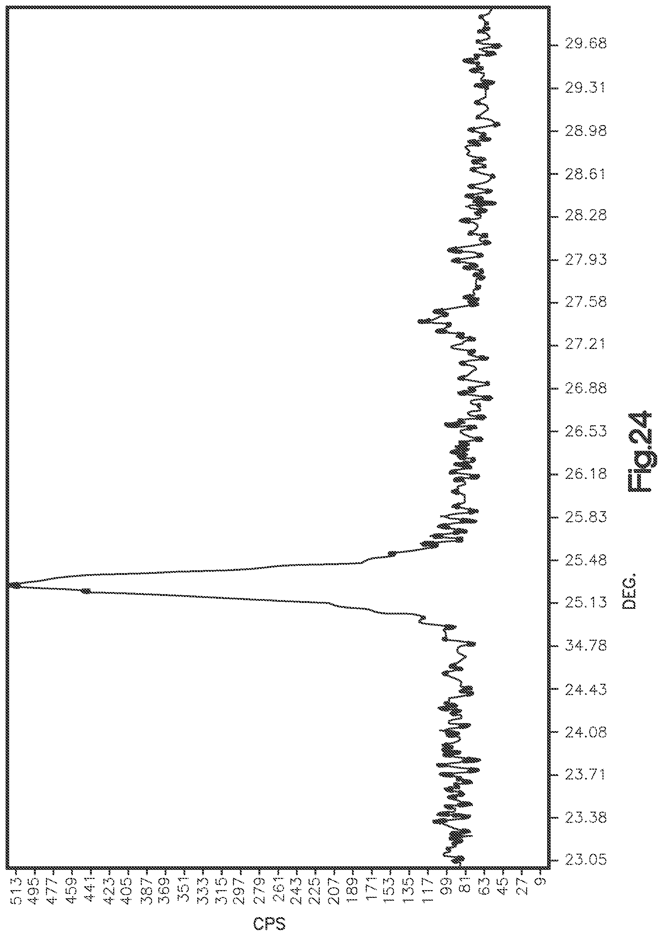

EBSD data is given in Figures 21-23. Figure 21 shows the SEM representation of the area being scanned. Figure 22 is a grain orientation map which shows the division of the amorphous and crystalline regions of the area scanned shown in Figure 21. Comparing Figures 21 and 22, the boundary between the smooth area and porous area can be distinguished as the boundary between the crystalline phase and amorphous phase. Furthermore, the different grain orientations found in the anatase and rutile crystalline area shows that the crystalline oxide is formed by many small different crystals formed on a single titanium grain. Figure 23 shows the distribution of the crystalline phases. Anatase was found to be the more prominent crystalline phase, as was to be expected from the XRD data (Figure 24). It should be understood that with EBSD testing the absence of the detection of a crystalline phase does not necessarily mean that the area is amorphous. Accordingly, the porous texture seen in Figure 20 and 21 is, without being bound to any particular theory, likely a highly crystalline area of anatase and rutile.

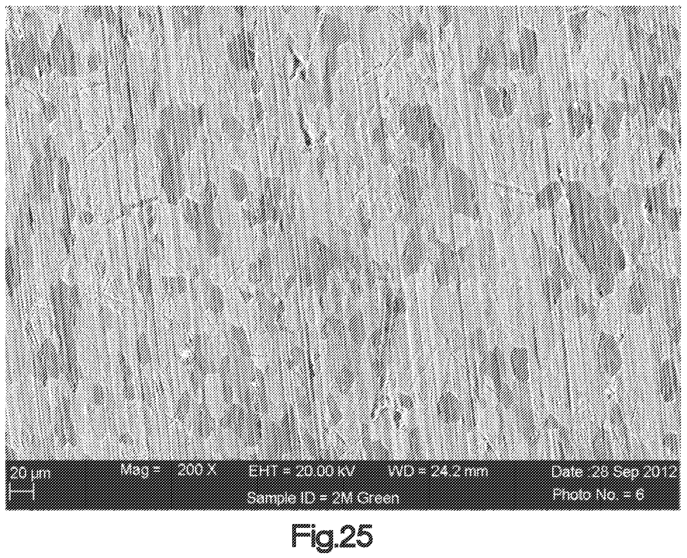

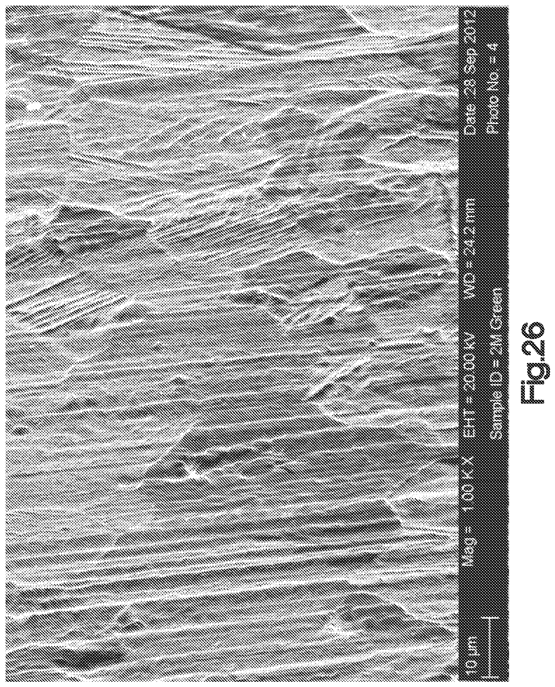

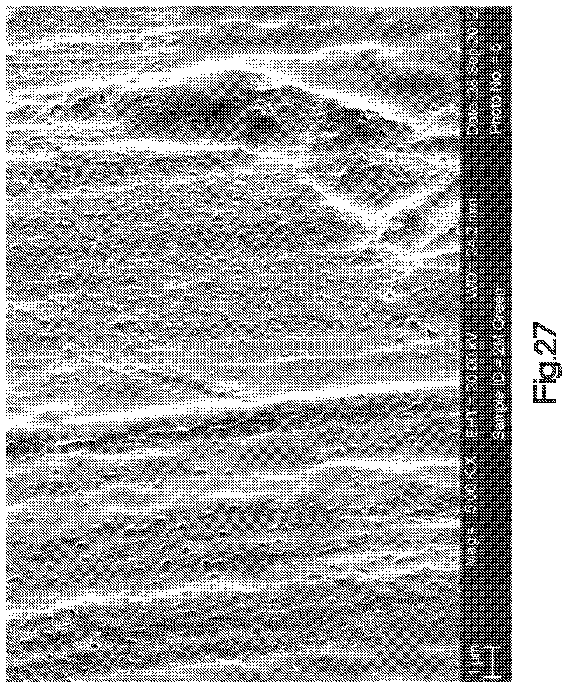

Figures 25-27 show the surface oxide of a green anodized titanium sample tested in 2.0 M sulfuric acid. The low magnification SEM image (Figure 25) shows a distribution of light and dark colored grains without any discernible surface texture differences similar to the 0.94 M green sample shown in Figure 21. Figure 26 shows a higher magnification (1000X) of the same area. The texture difference seen in the 0.94 M green sample cannot be as clearly seen in Figure 26. A higher magnification (5000X) image in Figure 27 shows that the boundaries that were evident in Figure 20 (0.94 M) are not as apparent in the 2.0 M sample. Further inspection shows that the textured areas still have micro porosity that is not found on the smooth areas. EBSD was used to evaluate a representative area at an approximate magnification of 15,000X.

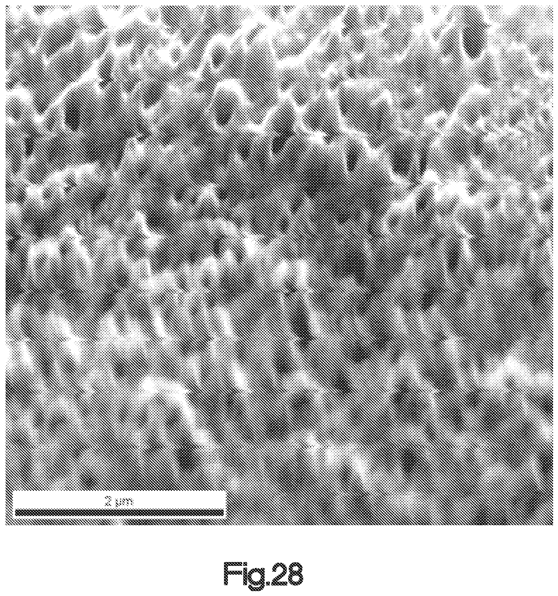

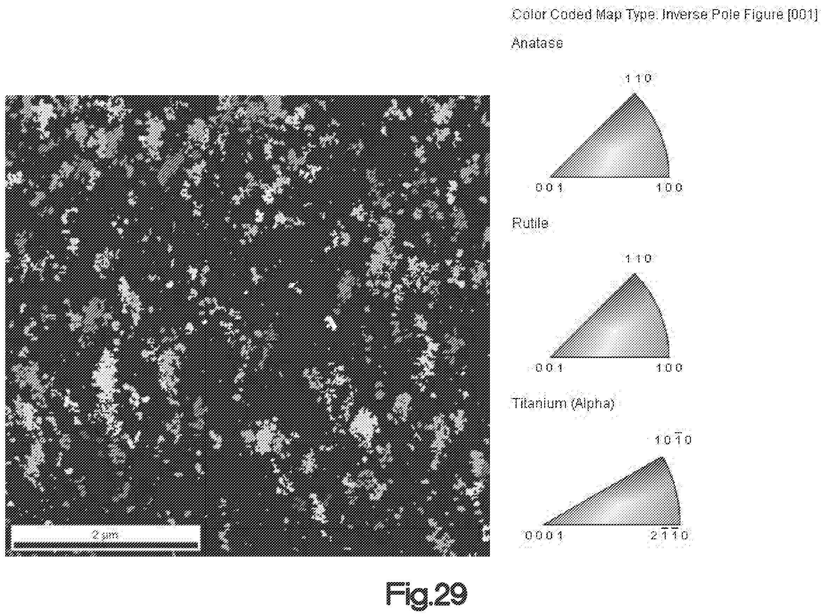

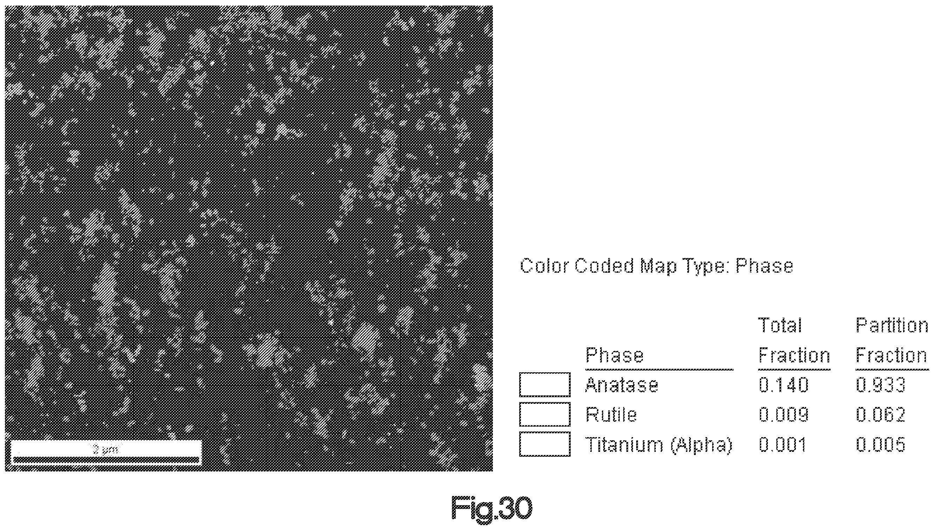

EBSD data is given in Figures 28-30. Figure 28 shows the SEM representation of the area being scanned. Figure 29 shows the amorphous and crystalline regions of the area scanned. Comparing Figures 28 and 29, there is no distinguishable boundary between the amorphous and crystalline areas of the surface oxide. The different grain orientations found in the crystalline area may show that the crystalline oxide is formed by many small different textured crystals. Figure 30 shows the distribution of the crystalline phases. Anatase was found to be the more prominent crystalline phase compared to rutile.

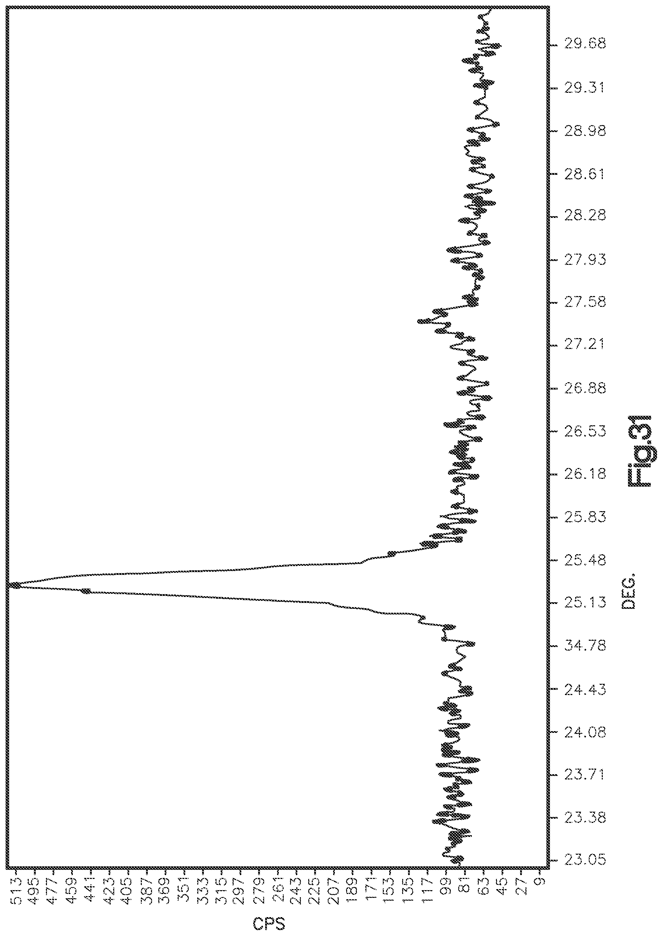

Comparing the SEM images for the gold anodized samples, the higher magnification images show a higher degree of the micro porosity surfaces for the 2 M compared to the 0.5 M sample. Without being bound to any particular theory, these areas may be the beginning of a crystalline oxide area being formed. The SEM and EBSD data from the 0.94 M and 2.0 M green samples shows a preliminary trend that as the molarity increases the confluence of the crystalline phase also increases. Comparing the X-Ray diffraction scans for both samples (Figures 24 and 31) indicates that the anatase peak heights for both samples are very similar. Without being bound to any particular theory, this may be an indicator that the crystalline phases (anatase and rutile) levels are similar for each thickness (color) but the confluence of the oxide may be influenced by the molarity of the anodization bath.

## Claims

1. A kit, comprising first and second medical devices, wherein each of the medical devices comprises: a substrate comprising titanium and a titanium oxide film surmounting at least a portion of the titanium,
at least a portion of the titanium oxide film being anatase phase, wherein the anatase phase is activated and comprises reactive oxygen species and hydroxyl radicals that provide antimicrobial properties, and
the titanium oxide film being of such a thickness selected to impart a specific visually perceptible color to the medical device according to the thickness of the titanium oxide film, wherein the first and second medical devices differ in the visually perceptible color.

2. The kit of claim 1, wherein the substrate comprises essentially pure titanium or a titanium alloy.

3. The kit of claim 1 or 2, further comprising a core underlying the substrate, wherein the core preferably comprises a polymer.

4. The kit of any one of claims 1 to 3, wherein the titanium oxide film is characterized as anodized.

5. The kit of any one of claims 1 to 4, wherein the thickness of the titanium oxide film is in the range of from about 20 nm to about 500 nm.

6. The kit of any one of claims 1 to 5, wherein the medical devices are configured as an implant, a needle, a catheter, or any combination thereof.

7. The kit of any one of claims 1 to 6, wherein the film comprises more than about 95% anatase titanium oxide.

8. The kit of any one of claims 1 to 7, wherein the substrate comprises an admixture of a polymer and titanium.

9. The kit of any one of claims 1 to 8, further comprising a source of light being operable to emit light of a wavelength and intensity sufficient to cause the titanium oxide to exhibit a biocidal effect upon irradiation with light from the light source, wherein the light preferably comprises a wavelength of between about 350 nm and about 380 nm.

10. The kit of any one of claims 1 to 9, further comprising a removable package that is essentially transparent to ultraviolet light, the first, second, or both devices being disposed within the removable package.

11. A method of processing a first medical device and a second medical device, the method comprising for each of the first and second medical devices the steps of:
contacting a substrate material comprising titanium with an electrolyte;
anodizing the substrate material so as to give rise to film of titanium oxide surmounting at least a portion of the substrate material, wherein the titanium oxide film is given rise to a thickness selected to impart a specific visually perceptible color to the respective medical device according to the thickness of the titanium oxide film, such that the first and second medical devices differ in the visually perceptible color; and
exposing the film to illumination having a wavelength in the range of between about 350 nm to about 380 nm to create reactive oxygen species and hydroxyl radicals that provide antimicrobial properties.

12. The method of claim 11, wherein the electrolyte comprises an acid, preferably sulfuric acid.

13. The method of claim 12, wherein the acid is between about 0.5 M to about 7.0 M.

14. The method of any one of claims 11 to 13, wherein the titanium oxide film has a thickness in the range of from about 20 nm to about 500 nm.

## Patentansprüche

1. Set, umfassend eine erste und zweite medizinische Vorrichtung, wobei jede der medizinischen Vorrichtungen umfasst:
ein Substrat, das Titan und einen Titanoxidfilm umfasst, der zumindest einen Teil des Titans überdeckt,
wobei zumindest ein Teil des Titanoxidfilms in der Anatas-Phase ist, wobei die Anatas-Phase aktiviert ist und reaktive Sauerstoffspezies und Hydroxyl-Radikale umfasst, die antimikrobielle Eigenschaften bereitstellen, und
wobei der Titanoxidfilm von einer solchen Dicke ist, die ausgewählt ist, der medizinischen Vorrichtung gemäß der Dicke des Titanoxidfilms eine spezifische visuell wahrnehmbare Farbe zu verleihen, wobei sich die erste und zweite medizinische Vorrichtung in der visuell wahrnehmbaren Farbe unterscheiden.

2. Set nach Anspruch 1, wobei das Substrat im Wesentlichen reines Titan oder eine Titanlegierung umfasst.

3. Set nach Anspruch 1 oder 2, des Weiteren umfassend einen Kern, der unter dem Substrat liegt, wobei der Kern vorzugsweise ein Polymer umfasst.

4. Set nach einem der Ansprüche 1 bis 3, wobei der Titanoxidfilm als anodisiert charakterisiert ist.

5. Set nach einem der Ansprüche 1 bis 4, wobei die Dicke des Titanoxidfilms im Bereich von ungefähr 20 nm bis ungefähr 500 nm ist.

6. Set nach einem der Ansprüche 1 bis 5, wobei die medizinischen Vorrichtungen als ein Implantat, eine Nadel, ein Katheter oder eine beliebige Kombination davon eingerichtet sind.

7. Set nach einem der Ansprüche 1 bis 6, wobei der Film mehr als 95 % Anatas-Titanoxid umfasst.

8. Set nach einem der Ansprüche 1 bis 7, wobei das Substrat eine Zumischung eines Polymers und Titan umfasst.

9. Set nach einem der Ansprüche 1 bis 8, des Weiteren umfassend eine Lichtquelle, die betriebsfähig ist, Licht einer Wellenlänge und Intensität auszustrahlen, ausreichend, um zu bewirken, dass der Titanoxidfilm bei Bestrahlung mit Licht von der Lichtquelle einen bioziden Effekt zeigt, wobei das Licht vorzugsweise eine Wellenlänge von zwischen ungefähr 350 nm und ungefähr 380 nm umfasst.

10. Set nach einem der Ansprüche 1 bis 9, des Weiteren umfassend eine entfernbare Verpackung, die für ultraviolettes Licht im Wesentlichen transparent ist, wobei die erste, zweite oder beide Vorrichtungen in der entfernbaren Verpackung angeordnet sind.

11. Verfahren zum Bearbeiten einer ersten medizinischen Vorrichtung und einer zweiten medizinischen Vorrichtung, wobei das Verfahren für jede der ersten und zweiten medizinischen Vorrichtung die Schritte umfasst:
Kontaktieren eines Titan umfassenden Substratmaterials mit einem Elektrolyt,
Anodisieren des Substratmaterials, um einen Film von Titanoxid zu bilden, der zumindest einen Teil des Substratmaterials überdeckt, wobei der Titanoxidfilm bis zu einer Dicke gebildet wird, die ausgewählt ist, der entsprechenden medizinischen Vorrichtung gemäß der Dicke des Titanoxidfilms eine spezifische visuell wahrnehmbare Farbe zu verleihen, so dass sich die erste und zweite medizinische Vorrichtung in der visuell wahrnehmbaren Farbe unterscheiden, und
Aussetzen des Films einer Bestrahlung mit einer Wellenlänge im Bereich von zwischen ungefähr 350 nm bis ungefähr 380 nm, um reaktive Sauerstoffspezies und Hydroxyl-Radikale zu erzeugen, die antimikrobielle Eigenschaften bereitstellen.

12. Verfahren nach Anspruch 11, wobei der Elektrolyt eine Säure, vorzugsweise Schwefelsäure, umfasst.

13. Verfahren nach Anspruch 12, wobei die Säure zwischen ungefähr 0,5 M bis ungefähr 7,0 M ist.

14. Verfahren nach einem der Ansprüche 11 bis 13, wobei der Titanoxidfilm eine Dicke im Bereich von ungefähr 20 nm bis ungefähr 500 nm aufweist.

## Revendications

1. Kit, comprenant des premier et second dispositifs médicaux, dans lequel chacun des dispositifs médicaux comprend :
un substrat comprenant du titane et un film en oxyde de titane surmontant au moins une portion du titane ;
au moins une portion du film d'oxyde de titane étant une phase anatase, dans lequel la phase anatase est activée et comprend des espèces réactives d'oxygène et des radicaux hydroxyle fournissant des propriétés antimicrobiennes, et
le film d'oxyde de titane étant d'une épaisseur sélectionnée de manière à conférer au dispositif médical une couleur spécifique perceptible visuellement en fonction de l'épaisseur du film d'oxyde de titane, dans lequel les premier et second dispositifs médicaux diffèrent de par la couleur perceptible visuellement.

2. Kit selon la revendication 1, dans lequel le substrat comprend du titane essentiellement pur ou un alliage de titane.

3. Kit selon la revendication 1 ou 2, comprenant en outre un noyau sous-jacent au substrat, dans lequel le noyau comprend de préférence un polymère.

4. Kit selon l'une quelconque des revendications 1 à 3, dans lequel le film d'oxyde de titane est caractérisé comme anodisé.

5. Kit selon l'une quelconque des revendications 1 à 4, dans lequel l'épaisseur du film d'oxyde de titane est comprise dans l'intervalle allant d'environ 20 nm à environ 500 nm.

6. Kit selon l'une quelconque des revendications 1 à 5, dans lequel les dispositifs médicaux sont configurés comme un implant, une aiguille, un cathéter, ou une combinaison de ceux-ci.

7. Kit selon l'une quelconque des revendications 1 à 6, dans lequel le film comprend plus d'environ 95 % d'oxyde de titane anatase.

8. Kit selon l'une quelconque des revendications 1 à 7, dans lequel le substrat comprend un mélange d'un polymère et de titane.

9. Kit selon l'une quelconque des revendications 1 à 8, comprenant en outre une source de lumière pouvant fonctionner pour émettre une lumière avec une longueur d'onde et une intensité suffisantes pour amener l'oxyde de titane à présenter un effet biocide suite à l'irradiation de lumière à partir de la source de lumière, dans lequel la lumière comprend de préférence une longueur d'onde s'étendant entre environ 350 nm et environ 380 nm.

10. Kit selon l'une quelconque des revendications 1 à 9, comprenant en outre un conditionnement amovible essentiellement transparent à la lumière ultraviolette, les premier, second ou les deux dispositifs étant disposés dans le conditionnement amovible.

11. Procédé de traitement d'un premier dispositif médical et d'un second dispositif médical, le procédé comprenant pour chacun des premier et second dispositifs médicaux les étapes consistant à :
mettre en contact un matériau de substrat comprenant du titane avec un électrolyte ;
anodiser le matériau de substrat de manière à générer un film en oxyde de titane surmontant au moins une portion du matériau de substrat, dans lequel le film d'oxyde de titane est généré avec une épaisseur sélectionnée de manière à conférer au dispositif médical respectif une couleur spécifique perceptible visuellement en fonction de l'épaisseur du film d'oxyde de titane, de telle sorte que les premier et second dispositifs médicaux diffèrent de par la couleur perceptible visuellement, et
exposer le film à un éclairage présentant une longueur d'onde comprise dans un intervalle s'étendant entre environ 350 nm et environ 380 nm afin de créer des espèces réactives d'oxygène et des radicaux hydroxyle fournissant des propriétés antimicrobiennes.

12. Procédé selon la revendication 11, dans lequel l'électrolyte comprend un acide, de préférence de l'acide sulfurique.

13. Procédé selon la revendication 12, dans lequel l'acide est compris entre environ 0,5 M et environ 7,0 M.

14. Procédé selon l'une quelconque des revendications 11 à 13, dans lequel le film d'oxyde de titane présente une épaisseur comprise dans l'intervalle allant d'environ 20 nm à environ 500 nm.
